# EUROPEAN PATENT SPECIFICATION

(11) **EP 2 327 754 B1**
(45) Date of publication and mention of the grant of the patent: **05.09.2018**
(21) Application number: 10014853.5
(22) Date of filing: 26.06.2006
(51) Int. Cl.: C09K 3/30, A61K 8/04, A61Q 19/00, A61Q 5/06, A61Q 15/00, A61K 9/00

(54) **Compositions containing fluorine substituted olefins**
Zusammensetzungen mit fluorsubstituierten Olefinen
Compositions contenant des oléfines substituées par du fluor

(30) Priority: 24.06.2005 US 693853 P
(43) Date of publication of application: 01.06.2011
(62) Divisional of application: 06785612.0
(73) Proprietor: Honeywell International Inc., Morris Plains, NJ 07950 (US)
(72) Inventor: Pham, Hang T., Morristown, NJ 07962-2245 (US); Singh, Rajiv R., Morristown, NJ 07962-2245 (US)
(74) Representative: Crooks, Elizabeth Caroline

(56) References cited:
- US-A- 3 884 828
- US-A1- 2004 119 047
- US-A1- 2004 127 383
- US-A1- 2004 256 594
- US-A1- 2005 096 246
- US-B1- 6 300 378

## Description

### FIELD OF THE INVENTION

The present invention is directed to a system, container or device comprising a composition which comprises 1-chloro-3,3,3-trifluoropropene (HCFO-1233zd).

### BACKGROUND

Fluorocarbon based fluids have found widespread use in many commercial and industrial applications, including as the working fluid in systems such as air conditioning, heat pump and refrigeration systems, as aerosol propellants, as blowing agents, as heat transfer media, and as gaseous dielectrics. Because of certain suspected environmental problems, including the relatively high global warming potentials, associated with the use of some of the compositions that have heretofore been used in these applications, it has become increasingly desirable to use fluids having low or even zero ozone depletion potential, such as hydrofluorocarbons ("HFCs"). Thus, the use of fluids that do not contain chlorofluorocarbons ("CFCs") or hydrochlorofluorocarbons ("HCFCs") is desirable. Furthermore, some HFC fluids may have relatively high global warming potentials associated therewith, and it is desirable to use hydrofluorocarbon or other fluorinated fluids having as low global warming potentials as possible while maintaining the desired performance in use properties. Additionally, the use of single component fluids or azeotrope-like mixtures, which do not substantially fractionate on boiling and evaporation, is desirable in certain circumstances.

Certain fluorocarbons have been a preferred component in many heat exchange fluids, such as refrigerants, for many years in many applications. For, example, fluoroalkanes, such as chlorofluoromethane and chlorofluoroethane derivatives, have gained widespread use as refrigerants in applications including air conditioning and heat pump applications owing to their unique combination of chemical and physical properties. Many of the refrigerants commonly utilized in vapor compression systems are either single components fluids or azeotropic mixtures.

As suggested above, concern has been increasing in recent years about potential damage to the earth's atmosphere and climate, and certain chlorine-based compounds have been identified as particularly problematic in this regard. The use of chlorine-containing compositions (such as chlorofluorocarbons (CFC's), hydrochlorofluorocarbons (HCF's) and the like) as the working fluid in heat transfer systems, such as in refrigeration and air-conditioning systems, has become disfavored because of the ozone-depleting properties associated with many of such compounds. There has thus been an increasing need for new fluorocarbon and hydrofluorocarbon compounds and compositions that are attractive alternatives to the compositions heretofore used in these and other applications. For example, it has become desirable to retrofit chlorine-containing refrigeration systems by replacing chlorine-containing refrigerants with non-chlorine-containing refrigerant compounds that will not deplete the ozone layer, such as hydrofluorocarbons (HFC's). Industry in general and the heat transfer industry in particular are continually seeking new fluorocarbon based mixtures that offer alternatives to, and are considered environmentally safer substitutes for, CFCs and HCFCs. It is generally considered important, however, at least with respect to heat transfer fluids, that any potential substitute must also possess those properties present in many of the most widely used fluids, such as excellent heat transfer properties, chemical stability, low- or no- toxicity, non-flammability and/or lubricant compatibility, among others.

Applicants have come to appreciate that lubricant compatibility is of particular importance in many of applications. More particularly, it is highly desirably for refrigeration fluids to be compatible with the lubricant utilized in the compressor unit, used in most refrigeration systems. Unfortunately, many non-chlorine-containing refrigeration fluids, including HFC's, are relatively insoluble and/or immiscible in the types of lubricants used traditionally with CFC's and HFC's, including, for example, mineral oils, alkylbenzenes or poly(alpha-olefins). In order for a refrigeration fluid-lubricant combination to work at a desirable level of efficiently within a compression refrigeration, air-conditioning and/or heat pump system, the lubricant should be sufficiently soluble in the refrigeration liquid over a wide range of operating temperatures. Such solubility lowers the viscosity of the lubricant and allows it to flow more easily throughout the system. In the absence of such solubility, lubricants tend to become lodged in the coils of the evaporator of the refrigeration, air-conditioning or heat pump system, as well as other parts of the system, and thus reduce the system efficiency.

With regard to efficiency In use, it is important to note that a loss in refrigerant thermodynamic performance or energy efficiency may have secondary environmental impacts through increased fossil fuel usage arising from an increased demand for electrical energy.

Furthermore, it is generally considered desirably for CFC refrigerant substitutes to be effective without major engineering changes to conventional vapor compression technology currently used with CFC refrigerants.

Flammability is another important property for many applications. That is, it is considered either important or essential in many applications, including particularly in heat transfer applications, to use compositions which are non-flammable. Thus, it is frequently beneficial to use in such compositions compounds which are nonflammable. As used herein, the term "nonflammable" refers to compounds or compositions which are determined to be nonflammable as determined in accordance with ASTM standard E-681, dated 2002, which is incorporated herein by reference. Unfortunately, many HFC's which might otherwise be desirable for used in refrigerant compositions are not nonflammable. For example, the fluoroalkane difluoroethane (HFC-152a) and the fluoroalkene 1,1,1-trifluorpropene (HFO-1243zf) are each flammable and therefore not viable for use In many applications.

Higher fluoroalkenes, that is fluorine-substituted alkenes having at least five carbon atoms, have been suggested for use as refrigerants. U.S. Patent No. 4,788,352 - Smutny is directed to production of fluorinated C₅ to C₈ compounds having at least some degree of unsaturation. The Smutny patent identifies such higher olefins as being known to have utility as refrigerants, pesticides, dielectric fluids, heat transfer fluids, solvents, and intermediates in various chemical reactions. (See column 1, lines 11 - 22).

While the fluorinated olefins described in Smutny may have some level of effectiveness in heat transfer applications, it is believed that such compounds may also have certain disadvantages. For example, some of these compounds may tend to attack substrates, particularly general-purpose plastics such as acrylic resins and ABS resins. Furthermore, the higher olefinic compounds described in Smutny may also be undesirable in certain applications because of the potential level of toxicity of such compounds which may arise as a result of pesticide activity noted in Smutny. Also, such compounds may have a boiling point which is too high to make them useful as a refrigerant in certain applications.

Bromofluoromethane and bromochlorofluoromethane derivatives, particularly bromotrifluoromethane (Halon 1301) and bromochlorodifluoromethane (Halon 1211) have gained widespread use as fire extinguishing agents in enclosed areas such as airplane cabins and computer rooms. However, the use of various halons is being phased out due to their high ozone depletion. Moreover, as halons are frequently used in areas where humans are present, suitable replacements must also be safe to humans at concentrations necessary to suppress or extinguish fire.

US 2004/0256594 and US 2004/0119047 disclose compositions comprising a compound of Formula (I):

XCF_{z}R_{3-z} (I)

where X is a C2 or C3 unsaturated, substituted or unsubstituted alkyl radical, R is independently Cl, F Br, I or H, and z is 1 to 3, and the use of these compositions as refrigerants for heating and cooling, blowing agents, aerosol propellants, solvent compositions, and fire extinguishing and suppressing agents.

US 2004/0127383 discloses azeotrope-like compositions comprising a pentafluoropropene (HFO-1225), and a fluid selected from the group consisting of 3,3,3-trifluoropropene (HFO-1243zf), 1,1-difluoroethane (HFC-152a), trans-1,3,3,3-tetrafluoropropene (trans-HFO-1234ze), and combinations of two or more thereof, and the use of these compositions as refrigerants, blowing agents, sprayable compositions, and flame suppressants.

US 3884828 describes aerosol propellants and refrigerants based on trifluoropropene (CF₃CH=CH₂).

US 6300378 discloses compositions comprising bromine-containing halocarbon additives which are used to decrease or eliminate the flammability of aerosol propellants, refrigerants, foam blowing agents, solvents and sterilants.

US 2005/0096246 discloses compositions comprising a fluoroalkene containing from 3 to 4 carbon atoms and at least one carbon-carbon double bond, such as 1,2-dichloro-3,3,3-trifluoropropene (HFO-1223xd), which are indicated as being useful in solvent cleaning applications.

Applicants have thus come to appreciate a need for compositions that are useful in a system, container or device which is configured to propel or move an object, while avoiding one or more of the disadvantages noted above.

### SUMMARY

The present invention relates to a system, container or device comprising a composition comprising 1-chloro-3,3,3-trifluoropropene (HCFO-1233zd), and which is configured to propel or move an object.

Disclosed herein is the trifluoro,monochloropropene (HFCO-1233) class of compounds which includes CF₃CCl=CH₂ (HFO-1233xf) and OF₃CH=CHCl (HFO-1233zd).

The term "HFO-1233" is used herein to refer to all trifluoro,monochloropropenes. Among the trifluoromonochloropropenes are included 1,1,1,trifluoro-2,chloro-propene (HFCO-1233xf), both cis- and trans-1,1,1-trifluo-3,chlororopropene (HFCO-1233zd). The term HFCO-1233zd is used herein generically to refer to 1,1,1-trifluo-3,chloro-propene, independent of whether it is the cis- or trans- form. The terms "cisHFCO-1233zd" and "transHFCO-1233zd" are used herein to describe the cis- and trans- forms of 1, 1, 1-trifluo,3-chlororopropene, respectively. The term "HFCO-1233zd" therefore includes within its scope cisHFCO-1233zd, transHFCO-1233zd, and all combinations and mixtures of these.

### DETAILED DESCRIPTION OF PREFERRED EMBODIMENTS

### THE COMPOSITIONS

The preferred embodiments of the present invention are directed to a system, container or device comprising a composition comprising 1-chloro-3,3,3-trifluoropropene (HCFO-1233zd).

Applicants believe that, in general, HCFO-1233zd finds use in aerosols.

However, applicants have surprisingly and unexpectedly found that certain of the compounds described above exhibit a highly desirable low level of toxicity compared to other of such compounds. As can be readily appreciated, this discovery is of potentially enormous advantage and benefit for the formulation of not only refrigerant compositions, but also any and all compositions which would otherwise contain relatively toxic compounds satisfying the formulas described above.

The present compositions are believed to possess properties that are advantageous for a number of important reasons. For example, applicants believe, based at least in part on mathematical modeling, that the fluoroolefins of the present invention will not have a substantial negative affect on atmospheric chemistry, being negligible contributors to ozone depletion in comparison to some other halogenated species. The preferred compositions of the present invention thus have the advantage of not contributing substantially to ozone depletion. The preferred compositions also do not contribute substantially to global warming compared to many of the hydrofluoroalkanes presently in use.

Of course other compounds and/or components that modulate a particular property of the compositions (such as cost for example) may also be included in the present compositions, and the presence of all such compounds and components is within the broad scope of the invention.

In certain preferred forms, compositions of the present invention have a Global Warming Potential (GWP) of not greater than about 1000, more preferably not greater than about 500, and even more preferably not greater than about 150. In certain embodiments, the GWP of the present compositions is not greater than about 100 and even more preferably not greater than about 75. As used herein, "GWP" is measured relative to that of carbon dioxide and over a 100 year time horizon, as defined in "The Scientific Assessment of Ozone Depletion, 2002, a report of the World Meteorological Association's Global Ozone Research and Monitoring Project,"

In certain preferred forms, the present compositions also preferably have an Ozone Depletion Potential (ODP) of not greater than 0.05, more preferably not greater than 0.02 and even more preferably about zero. As used herein, "ODP" is as defined in "The Scientific Assessment of Ozone Depletion, 2002, A report of the World Meteorological Association's Global Ozone Research and Monitoring Project,".

The amount of the compounds, contained in the present compositions can vary widely, depending the particular application, and compositions containing more than trace amounts and less than 1 00% of the compound are within broad the scope of the present invention. Moreover, the compositions of the present invention can be azeotropic, azeotrope-like or non-azeotropic. In preferred embodiments, the present compositions comprise HCFO-1233zd in amounts from about 5% by weight to about 99% by weight, and even more preferably from about 5% to about 95%. Many additional compounds or components, including lubricants, stabilizers, metal passivators, corrosion inhibitors, flammability suppressants, and other compounds and/or components that modulate a particular property of the compositions (such as cost for example) may be included in the present compositions, and the presence of all such compounds and components is within the broad scope of the invention.

The relative amount of any additional components which may be included in present compositions, can vary widely within the general broad scope of the present invention according to the particular application for the composition, and all such relative amounts are considered to be within the scope hereof.

Accordingly, applicants have recognized that the compositions of the present invention can be used to great advantage in sprayable composition applications and aerosol applications.

It is believed that those of skill in the art will be readily able to adapt the present compositions for use in any and all such applications without undue experimentation.

### PROPELLANT AND AEROSOL COMPOSITIONS

Disclosed herein are propellant compositions comprising or consisting essentially of a composition of the present invention.

The propellant composition is preferably a sprayable composition, either alone or in combination with other known propellants.

The present compositions may be used for propelling objects, including solid and/or liquid objects and/or gaseous objects, by applying to such objects a force generated by the present composition, such as would occur through the expansion of the compositions. For example, such force may preferably be provided, at least in part, by the change of phase of the compositions

from liquid to gas, and/or by the force released as a result of a substantial pressure reduction as the composition exits from a pressurized container. In this way, the compositions may be used to apply a burst of force, or a sustained force to an object to be propelled. Accordingly, the present invention relates to systems, containers and devices which include compositions comprising HCFO-1233zd and which are configured to propel or move an object, either a liquid object or a solid object or a gaseous object, with the desired amount of force. Examples of such uses include containers (such as pressurized cans and similar devices) which may be used, through the propellant force, to unblock drains, pipes or blockages in conduits, channels or nozzles.

Aerosol products for industrial, consumer or medical use typically contain one or more propellants along with one or more active ingredients, inert ingredients or solvents. The propellant provides the force that expels the product in aerosolized form. While some aerosol products are propelled with compressed gases like carbon dioxide, nitrogen, nitrous oxide and even air, most commercial aerosols use liquefied gas propellants. The most commonly used liquefied gas propellants are hydrocarbons such as butane, isobutane, and propane. Dimethyl ether and HFC-152a (1, 1-difluoroethane) are also used, either alone or in blends with the hydrocarbon propellants. Unfortunately, all of these liquefied gas propellants are highly flammable and their incorporation into aerosol formulations will often result in flammable aerosol products.

Applicants have come to appreciate the continuing need for nonflammable, liquefied gas propellants with which to formulate aerosol products. The present invention provides compositions comprising HCFO-1233zd for use in certain industrial aerosol products, including for example spray cleaners, lubricants, and the like, and in medicinal aerosols, including for example to deliver medications to the lungs or mucosal membranes. Examples of this includes metered dose inhalers (MDIs) for the treatment of asthma and other chronic obstructive pulmonary diseases and for delivery of medicaments to accessible mucous membranes or intranasally. The present disclosure describes methods for treating ailments, diseases and similar health related problems of an organism (such as a human or animal) comprising applying a composition of the present invention containing a medicament or other therapeutic component to the organism in need of treatment.

The step of applying the present composition may comprise providing a MDI containing the composition of the present invention (for example, introducing the composition into the MDI) and then discharging the present composition from the MDI.

The compositions of the present invention are capable of providing nonflammable, liquefied gas propellant and aerosols that do not contribute substantially to global warming. The present compositions can be used to formulate a variety of industrial aerosols or other sprayable compositions such as contact cleaners, dusters, lubricant sprays, and the like, and consumer aerosols such as personal care products, household products and automotive products.

The medicinal aerosol and/or propellant and/or sprayable compositions of the present invention in many applications include, in addition to HCFO-1233zd, a medicament such as a beta-agonist, a corticosteroid or other medicament, and, optionally, other ingredients, such as surfactants, solvents, other propellants, flavorants and other excipients. The compositions of the present invention, unlike many compositions previously used in these applications, have good environmental properties and are not considered to be potential contributors to global warming. The present compositions therefore provide in certain preferred embodiments substantially nonflammable, liquefied gas propellants having very low Global Warming potentials.

## Claims

1. A system, container or device which comprises a composition comprising 1-chloro-3,3,3-trifluoropropene (HCFO-1233zd), and which is configured to propel or move an object.

2. The system, container or device of claim 1, wherein the object is selected from a liquid, solid or gaseous object.

3. The system, container or device of claim 1 or 2, wherein the HCFO-1233zd is cis-HCFO-1233zd, trans-HCFO-1233zd or combinations thereof.

4. The system, container or device of claim 1 or 2, wherein the HCFO-1233zd is trans-HCFO-1233zd.

5. The system, container or device of any preceding claim, said composition comprising from 5 to 99% by weight of HCFO-1233zd.

6. The system, container or device of any preceding claim, said composition comprising from 5 to 95% by weight of HCFO-1233zd.

7. The system, container or device of any preceding claim, said composition having a Global Warming Potential (GWP) of not greater than 1000.

8. The system, container or device of any preceding claim, said composition having a GWP of not greater than 500.

9. The system, container or device of any preceding claim, said composition having a GWP of not greater than 150.

10. The system, container or device of any preceding claim, said composition having an Ozone Depletion Potential (ODP) of not greater than 0.02.

11. The system, container or device of any preceding claim, in the form of an industrial aerosol.

12. The system, container or device of claim 11, wherein the industrial aerosol is selected from a contact cleaner, duster or lubricant spray.

13. The system, container or device of any of claims 1 to 10, in the form of a consumer aerosol.

14. The system, container or device of claim 13, wherein the consumer aerosol is selected from a personal care product, household product or automotive product.

15. The system, container or device of any of claims 1 to 10, in the form of a medicinal aerosol, and further comprising a medicament.

16. The system, container or device of claim 15, wherein the medicament is selected from a beta agonist or corticosteroid.

## Patentansprüche

1. System, Behälter oder Vorrichtung, das bzw. der bzw. die eine Zusammensetzung, die 1-Chlor-3,3,3-trifluorpropen (HCFO-1233zd) umfasst und zum Antreiben oder Bewegen eines Objekts ausgebildet ist.

2. System, Behälter oder Vorrichtung nach Anspruch 1, wobei das Subjekt aus einem flüssigen, festen oder gasförmigen Objekt ausgewählt ist.

3. System, Behälter oder Vorrichtung nach Anspruch 1 oder 2, wobei es sich bei dem HCFO-1233zd um cis-HCFO-1233zd, trans-HCFO-1233zd oder Kombinationen davon handelt.

4. System, Behälter oder Vorrichtung nach Anspruch 1 oder 2, wobei es sich bei dem HCFO-1233zd um trans-HCFO-1233zd handelt.

5. System, Behälter oder Vorrichtung nach einem der vorhergehenden Ansprüche, wobei die Zusammensetzung 5 bis 99 Gew.-% HCFO-1233zd umfasst.

6. System, Behälter oder Vorrichtung nach einem der vorhergehenden Ansprüche, wobei die Zusammensetzung 5 bis 95 Gew.-% HCFO-1233zd umfasst.

7. System, Behälter oder Vorrichtung nach einem der vorhergehenden Ansprüche, wobei die Zusammensetzung ein Treibhauspotential (Global Warming Potential, GWP) von höchstens 1000 aufweist.

8. System, Behälter oder Vorrichtung nach einem der vorhergehenden Ansprüche, wobei die Zusammensetzung ein GWP von höchstens 500 aufweist.

9. System, Behälter oder Vorrichtung nach einem der vorhergehenden Ansprüche, wobei die Zusammensetzung ein GWP von höchstens 150 aufweist.

10. System, Behälter oder Vorrichtung nach einem der vorhergehenden Ansprüche, wobei die Zusammensetzung ein Ozonschädigungspotential (Ozone Depletion Potential, ODP) von höchstens 0,02 aufweist.

11. System, Behälter oder Vorrichtung nach einem der vorhergehenden Ansprüche in Form eines technischen Aerosols.

12. System, Behälter oder Vorrichtung nach Anspruch 11, wobei das technische Aerosol aus einem Kontaktreiniger, Verstäuber oder Schmiermittelspray ausgewählt ist.

13. System, Behälter oder Vorrichtung nach einem der Ansprüche 1 bis 10 in Form eines Verbraucher-Aerosols.

14. System, Behälter oder Vorrichtung nach Anspruch 13, wobei das Verbraucher-Aerosol aus einem Körperpflegeprodukt, Haushaltsprodukt oder Autoprodukt ausgewählt ist.

15. System, Behälter oder Vorrichtung nach einem der Ansprüche 1 bis 10 in Form eines medizinischen Aerosols und ferner umfassend ein Medikament.

16. System, Behälter oder Vorrichtung nach Anspruch 15, wobei das Medikament aus einem Beta-Agonisten oder Corticosteroid ausgewählt ist.

## Revendications

1. Système, récipient ou dispositif qui comprend une composition comprenant du 1-chloro-3,3,3-trifluoropropène (HCFO-1233zd) et qui est conçu pour propulser ou déplacer un objet.

2. Système, récipient ou dispositif selon la revendication 1, dans lequel l'objet est choisi entre un objet liquide, un objet solide et un objet gazeux.

3. Système, récipient ou dispositif selon la revendication 1 ou 2, dans lequel le HCFO-1233zd est le cis-HCFO-1233zd, le trans-HCFO-1233zd ou des associations de ceux-ci.

4. Système, récipient ou dispositif selon la revendication 1 ou 2, dans lequel le HCFO-1233zd est le trans-HCFO-1233zd.

5. Système, récipient ou dispositif selon une quelconque revendication précédente, ladite composition comprenant de 5 à 99 % en poids de HCFO-1233zd.

6. Système, récipient ou dispositif selon une quelconque revendication précédente, ladite composition comprenant de 5 à 95 % en poids de HCFO-1233zd.

7. Système, récipient ou dispositif selon une quelconque revendication précédente, ladite composition ayant un potentiel de réchauffement global (PRG) de pas plus de 1000.

8. Système, récipient ou dispositif selon une quelconque revendication précédente, ladite composition ayant un PRG de pas plus de 500.

9. Système, récipient ou dispositif selon une quelconque revendication précédente, ladite composition ayant un PRG de pas plus de 150.

10. Système, récipient ou dispositif selon une quelconque revendication précédente, ladite composition ayant un potentiel d'appauvrissement de la couche d'ozone (PACO) de pas plus de 0,02.

11. Système, récipient ou dispositif selon une quelconque revendication précédente, sous la forme d'un aérosol industriel.

12. Système, récipient ou dispositif selon la revendication 11, l'aérosol industriel étant choisi entre un nettoyant pour contacts, un dépoussiéreur et un vaporisateur de lubrifiant.

13. Système, récipient ou dispositif selon l'une quelconque des revendications 1 à 10, sous la forme d'un aérosol de grande consommation.

14. Système, récipient ou dispositif selon la revendication 13, l'aérosol de grande consommation étant choisi entre un produit de soins personnels, un produit ménager et un produit pour automobile.

15. Système, récipient ou dispositif selon l'une quelconque des revendications 1 à 10, sous la forme d'un aérosol médicinal et comprenant en outre un médicament.

16. Système, récipient ou dispositif selon la revendication 15, dans lequel le médicament est choisi entre un bêta-agoniste et un corticostéroïde.
